# EUROPEAN PATENT APPLICATION

(11) **EP 0 983 990 A2**
(43) Date of publication of application: **08.03.2000**
(21) Application number: 99810685.0
(22) Date of filing: 29.07.1999
(51) Int. Cl.: C07C 49/84, C07C 69/145, C07C 69/712, C07C 69/74, C07C 69/675, C07C 69/24, C07C 69/96, C07C 229/22, C07C 329/06, C11D 3/50, C11D 3/00, A61K 7/46

(54) **Ketones useful as precursors for organoleptic compounds**

(30) Priority: 04.09.1998 EP 98810883
(71) Applicant: Givaudan Roure (International) S.A., 1214 Vernier-Genève (CH)
(72) Inventor: Anderson, Denise Dr., 8032 Zürich (CH); Fráter, Georg Dr., 8400 Winterthur (CH)
(74) Representative: Patentanwälte Schaad, Balass, Menzl & Partner AG

(57) **Abstract**

The ketones of the formula I are useful for the delivery of organoleptic compounds, especially for flavours, fragrances and masking agents and antimicrobial compounds.

## Description

The present invention relates to new ketones. These ketones are useful as precursors for organoleptic compounds, especially for fragrances and masking agents and antimicrobial compounds.

A principal strategy currently employed in imparting odours to consumer products is the admixing of the fragrance directly into the product. There are, however, several drawbacks to this strategy. The fragrance material can be too volatile and or too soluble, resulting in fragrance loss during manufacturing, storage, and use. Many fragrance materials are also unstable over time. This again results in loss during storage.

In many consumer products it is desirable for the fragrance to be released slowly over time. Microencapsulation and inclusion complexes with cyclodextrins have been used to help decrease volatility, improve stability and provide slow-release properties. However, these methods are for a number of reasons often not successful. In additon, cyclodextrins can be too expensive.

Fragrance precursors for scenting fabrics being washed in the presence of a lipase-containing detergent are described in WO 95/04809. The fragrance precursors contained in the detergent and/or in the softener are cleaved by the lipase and a single odoriferous compound, either an odoriferous alcohol or ketone is yielded. Thereby a prolonged scenting effect on the fabric is obtained.

EP-A-0 816 322 describes fragrance precursor compositions containing carbonic acid esters or thiocarbonic acid esters.

These compositons are used in cosmetic products or in laundry products. The precursors produce fragrances upon contacting the skin or when used in the presence of lipases, e.g. as used in detergents, thus providing a prolongation of the fabric scenting effect.

One object of the present invention is to provide new precursors for compounds with different activities. A further object of the invention is to provide new compounds which are stable in a variety of applications including detergents, fabric softeners, cleaning products, personal care products, cosmetics, and sun protection products. A further object of the invention is to provide new compounds which can be cleaved by light, heat, hydrolysis and/or enzymes into one or more organoleptic compounds.

The present invention relates to new ketones of the formula I 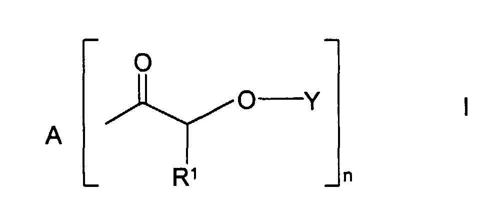 wherein
Y is an optionally substituted alkyl, cycloalkyl, cycloalkylalkyl, alkylcarbonyl, cycloalkylcarbonyl, arylcarbonyl, alkyloxycarbonyl, cycloalkyloxycarbonyl, aryloxycarbonyl, amide, oximinocarbonyl, alkylthiocarbonyl, cycloalkylthiocarbonyl, whereby all alkyl groups may be straight or branched and all alkyl and cycloalkyl groups may be saturated or unsaturated,
R¹ is hydrogen or a C₁₋₆ alkyl group which may be substituted, saturated or unsaturated, straight or branched,
A is a chromophoric aromatic or heteroaromatic ring or ring system,
A may carry 1 to 8 substituents selected from hydrogen, hydroxy, mercapto, amino, acyl, ester, esterified hydroxy group, cyano and nitro; alkyl, alkoxy, alkylthio and arylalkoxy which may be substituted, saturated or unsaturated, straight or branched; aryl and heteroaryl which may be substituted; one of the substituents may be a polymeric group, and
n is an integer
with the exception of
2-ethoxy-1-phenyl-ethanone,
hydroxy-acetic acid 2-oxo-2-phenyl-ethyl ester,
acetic acid 1-methyl-2-oxo-2-phenyl-ethyl ester, and
p-methoxyphenacyl acetate.

All groups containing the term alkyl include also the unsaturated residues, e.g. alkenyl, alkynyl, cycloalkenyl etc.

The compounds of the formula I are not limited to any particular stereoisomers, all possible stereoisomers (E/Z isomers, enantiomers, diastereomers) and all mixtures are thus included within the scope of the invention.

The compounds of the formula I are mostly or nearly odourless at room temperature, atmospheric conditions and about 20 to 100 % relative humidity. However, under activating conditions, they are cleaved and one or more active compounds with organoleptic properties are generated.

The activating conditions which lead to cleavage and the desired active compounds comprise the presence of light, such as room light and sunlight, sunlight being preferred. Additional activating conditions may be heat, hydrolysis and/or enzymes.

The compounds of the formula I yield after cleavage an aryl or heteroaryl ketone of the formula II

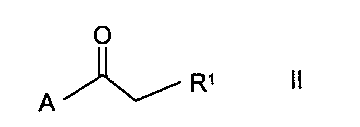

Preferred compounds are those which yield an organoleptic aryl ketone.

The compounds of the formula I, wherein Y is an alkyl, cycloalkyl or cycloalkylalkyl group, are cleaved into an aryl or heteroaryl ketone and an aldehyde or ketone, at least one thereof is organoleptic. Examples of organoleptic aryl ketones as well as of ketones and aldehydes including the preferred and more preferred ones are listed below. Examples of organoleptic heteroaryl ketones are 2-acetylpyrazine, 2-acetyl-3-methylpyrazine, 2-acetyl-5-ethylpyrazine, 2-acetylpyridine and the acetylpyridines which are disclosed in US patent 5,214,027.

The compounds of the formula I, wherein Y is an alkyl-, cycloalkyl- or aryloxycarbonyl group, are cleaved into an aryl or heteroaryl ketone and an alcohol, phenol, aldehyde or ketone, at least one thereof is organoleptic. Examples of alcohols and phenols including the preferred and more preferred ones are listed below.

The term phenol means an aromatic hydroxy compound wherein the hydroxy group is attached directly to the benzene ring.

The compounds of the formula I, wherein Y is an alkyl-, cycloalkyl- or arylcarbonyl group, yield after cleavage an aryl or heteroaryl ketone and an acid or a lactone, the aryl or heteroaryl ketone and/or the lactone being organoleptic. When the alkyl residue of the alkylcarbonyl group is substituted in alpha- or beta-position with a hydroxy group the released acid may be active as a peeling ingredient in cosmetics. Lactones may be formed when the alkyl residue is substituted in gamma- or delta-position with a hydroxy group or with the group O-CO-OR² or O-CO-R², wherein R² is an alkyl, aryl or aralkyl group which may be branched, unsaturated and/or substituted. Preferred compounds are those wherein R² is a C₁₋₃₀ alkyl group or a phenyl C₁₋₁₀ alkyl group. Compounds of the formula I with the group O-CO-OR² yield after cleavage besides the aryl or heteroaryl ketone and lactone an alcohol, phenol, aldehyde or ketone. The three obtained compounds can be organoleptic.

In the compounds of the formula I, wherein Y is an alkylcarbonyl group, the alkyl group may be substituted e.g. with an oxo group. These compounds yield after cleavage an aryl or heteroaryl ketone and a ketone, at least one thereof is organoleptic. Preferred compounds are those wherein the alkyl group is substituted in beta-position to the carbonyl group with the oxo group or in delta-position, in the latter case the oxo group may be situated in an optionally unsaturated cycloalkyl radical which may be substituted.

The compounds of the formula I, wherein Y is an amide, i.e. a N-substituted aminocarbonyl group, are cleaved into an aryl or heteroaryl ketone and an amine, at least one thereof is organoleptic. Examples of organoleptic amines including the preferred are listed below.

The compounds of the formula I, wherein Y is an alkylthiocarbonyl or cycloalkylthiocarbonyl group, are cleaved into an aryl or heteroaryl ketone and a thiol, at least one thereof is organoleptic. Examples of organoleptic thiols are 3,7-dimethyl-1-thio-octa-2,6-diene, 2-(1-mercapto-1-methylethyl)-5-methylcyclohexanone and 2-methoxy-2-methyl-butan2-thiol.

The compounds of the formula I, wherein Y is an oximinocarbonyl group, i.e. -CO-O-N=CR³R⁴, wherein R³ and R⁴ may be e.g. an optionally substituted, straight or branched, saturated or unsaturated alkyl group, are cleaved into an aryl or heteroaryl ketone and an oxime, at least one thereof is organoleptic.

The present invention includes also compounds of the formula I which are cleaved into an optionally organoleptic aryl or heteroaryl ketone and an organoleptic nitrile. In this case Y stands for an alkyl- or alkenylcarbonyl group which is substituted with a cyano group in alpha- and/or gamma-position to the carbonyl group, with the proviso that the chain between the carbonyl group and the cyano group has a double bond in beta-gamma-position to the carbonyl group when the cyano group stands in gamma-position. The alkyl or alkenyl group may be branched and/or substituted with further residues which may together form a ring system. Examples of organoleptic nitriles including the preferred ones are listed below.

In the compounds of the formula I A is a chromophoric aromatic or heteroaromatic ring or ring system. The term ring system includes mono- and polycyclic rings, which may be connected either directly or by a bridge, e.g. an optionally unsaturated alkylen group, oxygen etc., as well as fused rings. The heteroaromatic rings include five- and six-membered rings.

In most of the cases the obtained aryl or heteroaryl ketone is organoleptic. When one of the substituents of A represents a polymeric group or when A represents a fused aromatic or heteroaromatic ring system with more than two rings, the compounds of formula I release on cleavage a non-organoleptic aryl or heteroaryl ketone. In these cases O-Y in formula I represents a group which after cleavage yields at least an organoleptic aldehyde, an organoleptic ketone, an organoleptic alcohol or phenol, an organoleptic lactone, an organoleptic amine, an organoleptic thiol, an organoleptic oxime or an organoleptic nitrile, as described above.

Suitable polymeric groups are divinylbenzene-based polymers, ethyleneglycol dimethacrylate-based polymers, acrylic acid/maleic acid copolymers, acrylic acid homopolymers, polysiloxanes, alginic acid, carboxymethyl cellulose, xanthan gum and so on.

Since the compounds of the formula I, after cleavage, provide at least one substance of the group aryl or heteroaryl ketones, aldehydes, ketones, alcohols, phenols, amines, thiols, oximes, nitriles and lactones having organoleptic activity they permit the development of useful consumer products with enhanced organoleptic properties. The obtained organoleptic substances are useful as fragrances and masking agents and antimicrobial agents. Therefore, the present invention also relates to the use of all compounds of formula I as precursors for organoleptic compounds, in particular fragrances.

The compounds of the formula I can act as fragrance precursors in laundry products, such as detergents and fabric softeners, cleaning compositions, such as hard surface and all-purpose cleaners, personal care products, cosmetics or sun protection products. They can also act as precursors for odour masking agents in the same products as the fragrance precursors. They also can act as precursors for antimicrobial agents. The fragrance precursors and the precursors for odour masking agents may be used individually in an amount effective to enhance or to mask the characteristic odour of a material. More commonly the compounds are mixed with other fragrance components in an amount sufficient to provide the desired odour characteristics.

Due to the in situ generation of the active compounds the desired effect is prolonged and the substantivity on different substrates is enhanced. If two or more active compounds are provided, they can be generated, depending on the precursor simultaneously or successively. Further, the precursors of the invention provide slow release of the active compounds.

One preferred group of the ketones of the formula I are those wherein Y is an alkyl group. The alkyl group may contain a C₁₋₃₀ alkyl moiety, which may be unsaturated, branched and/or substituted by different groups as shown in the listed aldehydes and ketones below.

Preferred compounds of the formula I are those, wherein A is one aromatic ring and two of its substituents form a second ring, preferably a six-membered aromatic ring or a five- or six-membered aliphatic ring. Within this group of compounds it is most preferred that R¹ is hydrogen and the other sustituents of the aromatic ring are hydrogen or one or more methyl groups.

Preferred compounds of the formula I are also those, wherein R¹ is hydrogen or methyl. Preferably A carries no more than three substituents, especially methyl groups. A further preferred group of compounds of the formula I are those, wherein R¹ is hydrogen and A is one aromatic ring, which is unsubstituted or substituted with one methoxy group, preferably in the para-position.

When Y is an alkyloxycarbonyl group, C₁₋₃₀ alkyloxycarbonyl groups are preferred which may be unsaturated, branched and/or substituted by different groups as shown in the listed alcohols, aldehydes and ketones below. The aryloxycarbonyl group is preferably a phenyloxycarbonyl group which may be substituted as shown in the listed phenols. The cycloalkyloxycarbonyl group is preferably a C₃₋₈ cycloalkyloxycarbonyl group which may be unsaturated and/or substituted.

When Y is an alkylcarbonyl group, C₁₋₃₀ alkyl groups are preferred. They may be branched, unsaturated and/or substituted, e.g. by naphthol, phenoxy, naphthyloxy, hydroxy, amino, pyrocatechol, the group O-CO-OR² or O-CO-R², as stated above. When Y is a cycloalkylcarbonyl group, C₃₋₈ cycloalkyl moieties are preferred which can be unsaturated and/or substituted. When Y is an arylcarbonyl group, the phenylcarbonyl groups are preferred which may be substituted.

When Y is an amide (N-substituted aminocarbonyl) group a wide variety of non-organoleptic amines can be obtained by cleavage. For example, a list of suitable primary and secondary cosmetic amines can be found in the Cosmetic Ingredient Handbook edited by Joanne M. Nikitakis. Suitable surfactant amines can be found, for example, in Surfactants Europe edited by Gordon L. Hollis.

Preferred N-substituted aminocarbonyl groups are those which yield after cleavage the organoleptic amines listed below.

It is preferred that n is 1 or 2.

All groups for which Y stands may be substituted with one or more substituents selected from aryl; cycloalkyl; cycloalkenyl; oxo; oxy; cyano; hydroxy; hydroxy being esterified with a carboxylic acid, a carbonic acid ester, a N,N-substituted carbamic acid or an oximino carbonic acid; amino substituted with oximinocarbonyl; an esterified carboxylic group; and a carboxylic group esterified with an oxime.

The groups for which Y stands may also be substituted with the following substituents: A-CO-CHR¹-O-, A-CO-CHR¹-O-CO-, A-CO-CHR¹-O-alkoxy or A-CO-CHR¹-O-CO-alkoxy. The following formula III shows one example of this kind of compounds, wherein Y is an alkyl group substituted with A-CO-CHR¹-O-: 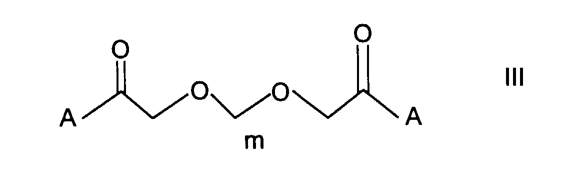 wherein m is an integer.

Compounds of the formula I may generate the following organoleptic aryl ketones:
4-methoxyphenyl-ethanone*
1-[6-(1,1-dimethylethyl)-2,3-dihydro-1,1-dimethyl-1H-inden- 4-yl]-ethanone
1-(5,6,7,8-tetrahydro-3,5,5,6,8,8-hexamethyl-2-naphthalenyl)-ethanone*
2-(1-methylethyl)-indanone*
4-tert-butyl-3,5-dinitro-2,6-dimethyl-acetophenone
1,6,7,8-tetrahydro-1,4,6,6,8,8-hexamethyl-as-indacen-3(2H)-one*
1-(2-napthalenyl)-ethanone*
1-(2,3-dihydro-1,1,2,3,3,6-hexamethyl-1H-inden-5-yl)-ethanone
1-[2,3-dihydro-1,1,2,6-tetramethyl-3-(1-methylethyl)-1H-inden-5-yl]-ethanone
3-methyl-1-(4-methylphenyl)-4-hexen-1-one
5-acetyl-1,1,2,3,3-pentamethylindane
1-phenylpropanone
acetophenone*
2,4-dimethylphenyl-ethanone*
1-[4-(1,1-dimethylethyl)-2,6-dimethylphenyl]-ethanone
1-(hexahydrodimethyl-1H-benzindenyl)-ethanone*
1-(5,6,7,8-tetrahydro-2-naphthalenyl)-ethanone
1-phenyl-4-penten-1-one
1-(5,6,7,8-tetrahydro-3,5,5,8,8-pentamethyl-2-naphthalenyl)-ethanone
1-(3-ethyl-5,6,7,8-tetrahydro-5,5,8,8-tetramethyl-2-naphthalenyl)-ethanone
whereby * indicates the preferred aryl ketones.

Compounds of the formula I may generate the following ketones:
2,5-dimethyl-oct-2-en-6-one**
4-(2,6,6-trimethylcyclohex-1-en-1-yl)butan-2-one**
4-(2,6,6-trimethylcyclohex-2-en-1-yl)butan-2-one**
2-methyl-5-(1-methylethenyl)-cyclohex-2-en-1-one*
1-(4-hydroxyphenyl)-butan-3-one**
4-benzo-1,3-dioxo-5-yl-but-2-one**
2-heptyl-cyclopentanone
nonan-2-one*
octan-2-one*
2,2,6,10-tetrametyltricyclo-[5.4.0.0(6,10)]-undecan-4-one
heptan-2-one*
undecan-2-one*
benzylacetone*
butan-2-one*
1,2,3,5,6,7-hexahydro-1,1,2,3,3,-pentamethyl-4H-inden-4-one*
6-methyl-hept-5-en-2-one*
2-(butan-2-yl)-cyclohexanone*
2-hexyl-cyclopent-2-en-1-one*
2-(1-methylethyl)-5-methyl-cyclohexanone*
2-(2-methylethyl)-5-methyl-cyclohexanone*
3-methyl-cyclopentadecanone
4-(1,1-dimethylpropyl)-cyclohexanone*
6,10-dimethyl-undeca-5,9-dien-2-one*
3-oxo-2-pentyl-cyclopentane-acetic acid methyl ester**
1-(1,2,3,4,5,6,7,8-octahydro-2,3,8,8-tetramethyl-2-naphthalenyl)-ethanone*
3-methyl-5-propyl-cyclohex-2-en-1-one*
1-(2-cyclohexen)-2,4,4-trimethyl-but-2-enone*
carvone**
2-hexyl-cyclo-pent-2-en-1-one**
2-pentyl-cyclopent-2-en-1-one
3-methyl-2-pentyl-cyclopent-2-en-1-one**
2-hexylidene-cyclopentanone*
3,5-diethyl-5,6-dimethyl-2-cyclohexenone*
4,4a,5,6,7,8-hexahydro-6-isopropenyl-4,4a-dimethyl-2(3H)-napthalenone**
3-methyl-6-propylidenecyclohexanone*
4-(1-methylethyl)-cyclohex-2-en-1-one
(E)-oct-3-en-2-one
1-(2,3,4,7,8,8A-hexahydro-3,6,8,8-tetramethyl-1H-3A,7-methanoazulen-5-yl)-ethanone*
2-hydroxy-3,5-dimethyl-cyclopent-2-ene-1-one*
1-(3,3-dimethyl-1-cyclohexen-1-yl)ethanone*
1-(2,4,6-trimethylcyclohex-3-en-1-yl)-but-1-en-3-one acetylisolongifolene
2-(3-methylbut-2-en-1-yl)-3-methyl-cyclopent-2-en-1-one
3-methyl-5-(2,2,3-trimethylcyclopent-3-en-1-yl)pent-3-en-2-one*
5-butylidene-2,2,4-trimethylcyclopentanone
1,2,3,5,6,7-hexahydro-1,1,2,3,3-pentamethyl-4H-inden-4-one**
3-methyl-5-propyl-cyclohex-2-en-1-one**
4,4a,5,6,7,8-hexahydro-6-isopropyl-2(3H)-naphthalenone
3,5,5-trimethyl-cyclohex-2-en-1,4-dione*
(E)-5-methyl-2-hepten-4-one
acetyl diisoamylene**
dec-3-en-2-one
2-ethyl-3,6,6-trimethylcyclohex-2-enyl-but-2-en-1-one
1-(5,5-dimethyl-1(6)-cyclohexen-1-yl)-4-penten-1-one**
1-(2,6,6-trimethyl-1-cyclohexen-1-yl)-but-2-en-1-one**
1-(2,6,6-trimethyl-2-cyclohexen-1-yl)-but-2-en-1-one**
1-(2,6,6,trimethyl-3-cyclohexen-1-yl)-but-2-en-1-one**
2,4,4,5,5-pentamethyl-1-cyclopentene-1-yl-ethanone*
whereby * indicates the preferred ketones and ** indicate the more preferred ketones.

Compounds of the formula I may generate the following aldehydes:
2,6,10-trimethylundec-9-enal*
1,2,3,4,5,6,7,8-octahydro-8,8-dimethyl-2-napthalenecarboxaldehyde
tridecanal
2-[4-(1-methylethyl)phenyl]-ethanal
2,4-dimethyl-cyclohex-3-ene-1-carboxaldehyde*
4-carboxaldehyde-1,3,5-trimethyl-cyclohex-1-ene*
1-carboxaldehyde-2,4-dimethyl-cyclohex-3-ene*
1-carboxaldehyde-4-(4-hydroxy-4-methylpentyl)-cyclohex-3-ene*
hex-2-enal*
3,5,5-trimethyl-hexanal
heptanal*
2,6-dimethyl-hept-5-eneal*
decanal**
dec-9-enal
dec-4-en-1-al
2-methyldecanal*
undec-10-ene-1-al**
undecanal*
dodecanal**
2-methyl-undecanal**
tridecanal
2-tridecenal
octanal**
nonanal*
3,5,5-trimethylhexanal
2-nonenal
undec-9-enal**
2-phenyl-propanal*
4-methyl-phenyl acetaldehyde*
3,7-dimethyl-octanal*
dihydrofarnesal**
7-hydroxy-3,7-dimethyl-octanal*
2,6-dimethyl-oct-5-ene-1-al
2-(4-(1-methylethyl)phenyl)-ethanal*
3-(3-isopropyl-phenyl)-butanal**
2-(3,7-dimethyl-oct-6-en-oxy)-ethanal
1-carboxaldehyde-4-(4-methyl-3-penten-1-yl)-cyclohex-3-ene*
2,3,5,5,-tetramethyl-hexanal
longifolic aldehyde
2-methyl-4-(2,6,6-trimethylcyclohex-2-en-1-yl)-butanal*
2-methyl-3-(4-tert-butylphenyl)propanal**
4-(1,1-dimethyl-ethyl)-benzenepropanal*
2-[4-(1-methyl-ethyl)phenyl]-propanal
alpha-methyl-1,3-benzodioxole-5-propanal*
3,7-dimethyl-oct-6-en-1-al*
2-methyl-3-(p-isopropylphenyl)-propionaldehyde*
4-(4-hydroxy-4-methyl-pentyl)-cyclohex-3-en-1-carboxaldehyde**
alpha-methyl-1,3-benzodioxole-5-propanal*
1-carboxaldehyde-4-(1,1-dimethylethyl)-cyclohexane
4-(octahydro-4,7-methano-5H-inden-5-ylidene)-butanal
[(3,7-dimethyl-6-octenyl)oxy]-acetaldehyde**
3,7-dimethyl-oct-2,6-dien-1-al*
nonadienal*
2,4-dimethyl-2,6-heptadienal
trans-dec-2-en-1-al*
2,4-diethyl-hep-2,6-dien-1-al*
dodec-2-en-1-al*
3,7-dimethyl-oct-2,6-dien-1-al*
2,4-diethyl-hepta-2,6-dienal
3,7-dimethyl-nona-2,6-dien-1-al*
3-propyl-2-hepten-1-al
1-carboxaldehyde-4-(prop-2-en-2-yl)-cyclohex-1-ene
whereby * indicates the preferred aldehydes and ** indicate the more preferred aldehydes.

Compounds of the formula I may generate the following alcohols and phenols:
amyl alcohol
hexyl alcohol*
2-hexyl alcohol*
heptyl alcohol*
octyl alcohol*
nonyl alcohol*
decyl alcohol*
undecyl alcohol*
lauryl alcohol*
myristic alcohol
3-methyl-but-2-en-1-ol*
3-methyl-1-pentanol
cis-3-hexenol*
cis-4-hexenol*
3,5,5-trimethyl-hexanol
3,4,5,6,6-pentamethylheptan-2-ol*
citronellol*
geraniol*
oct-1-en-3-ol
2,5,7-trimethyl-octan-3-ol
2-cis-3,7-dimethyl-2,6-octadien-1-ol
6-ethyl-3-methyl-5-octen-1-ol*
3,7-dimethyl-oct-3,6-dienol*
3,7-dimethyloctanol*
7-methoxy-3,7-dimethyl-octan-2-ol*
cis-6-nonenol*
5-ethyl-2-nonanol
6,8-dimethyl-2-nonanol*
2,2,8-trimethyl-7(8)-nonene-3-ol
nona-2,6-dien-1-ol
4-methyl-3-decen-5-ol*
dec-9-en-1-ol
benzylalcohol
2-methyl-undecanol
10-undecen-1-ol
1-phenyl-ethanol*
2-phenyl-ethanol*
2-methyl-3-phenyl-3-propenol
2-phenyl-propanol*
3-phenyl-propanol*
4-phenyl-2-butanol
2-methyl-5-phenyl pentanol*
2-methyl-4-phenyl-pentanol*
3-methyl-5-phenyl-pentanol*
2-(2-methylphenyl)-ethanol*
4-(1-methylethyl)-benzene methanol
4-(4-hydroxyphenyl)-butan-2-one*
2-phenoxy-ethanol*
4-(1-methylethyl)-2-hydroxy-1-methyl benzene
2-methoxy-4-methyl-phenol
4-methyl-phenol
anisic alcohol*
p-tolyl alcohol*
cinnamic alcohol*
vanillin*
ethyl vanillin*
eugenol*
isoeugenol*
thymol
anethol*
decahydro 2-naphthalenol
borneol*
cedrenol*
farnesol*
fenchyl alcohol*
menthol*
3,7,11-trimethyl-2,6,10-dodecatrien-1-ol
alpha ionol*
tetrahydro ionol*
2-(1,1-dimethylethyl)-cyclohexanol*
3-(1,1-dimethylethyl)-cyclohexanol*
4-(1,1-dimethylethyl)-cyclohexanol*
4-isopropyl cyclohexanol
6,6-dimethyl-bicyclo[3.3.1 ]hept-2-ene-2-ethanol
6,6-dimethyl-bicyclo[3.1.1 ]hept-2-ene-methanol*
p-menth-8-en-3-ol*
3,3,5-trimethyl-cyclohexanol
2,4,6-trimethyl-3-cyclohexenyl-methanol*
4-(1-methylethyl)-cyclohexane-methanol*
4-(1,1-dimethylethyl)-cyclohexanol
2-(1,1-dimethylethyl)-cyclohexanol
2,2,6-trimethyl-alpha-propyl cyclohexane propanol*
5-(2,2,3-trimethyl-3-cyclo-pentenyl)-3-methylpentan-2-ol*
3-methyl-5-(2,2,3-trimethylcyclopentyl-3-enyl)-pent-4-en-2-ol*
2-ethyl-4(2,2,3-trimethylcyclopentyl-3-enyl)-but-2-en-1-ol*
4-(5,5,6-trimethylbicyclo[2.2.1 ]hept-2-yl)-cyclohexanol*
2-(2-methylpropyl)-4-hydroxy-4-methyl-tetrahydropyran*
2-cyclohexyl propanol*
2-(1,1-dimethylethyl)-4-methyl-cyclohexanol*
1-(2-tert-butyl-cyclohexyloxy)-2-butanol*
1-(4-isopropyl-cyclohexyl)-ethanol*
1-(4-hydroxyphenyl)-butan-3-one
2,6-dimethyl-oct-7-en-2-ol*
2,6-dimethyl-heptan-2-ol*
3,7-dimethyl-octa-1,6-dien-3-ol*
whereby * indicates preferred alcohols and phenols.

Compounds of the formula I may generate the following organoleptic amines:
anthranilic acid 1-methyl-1-(4-methyl-3-cyclohexen-1-yl)ethyl ester
benzopyrrole
8,8-di(1H-indol-1-yl)-2,6-dimethyl-octane-2-ol
anthranilic acid allyl ester
anthranilic acid 1,5-dimethyl-1-vinyl-4-hexenyl ester
2-amino-benzoic acid methyl ester*
methyl anthranilic acid N-(2-methylpent-1-en-1-yl) ester
1-methyl-1-(4-methyl-3-cyclohexen-1-yl)ethyl anthranilic acid
anthranilic acid phenylethyl ester*
2-methylamino-benzoic acid methyl ester*
6-methyltetrahydro-quinoline
isobutyl N-methyl anthranilate
(Z)-3-hexenyl-2-aminobenzoate*
whereby * indicates the preferred organoleptic amines.

Compounds of formula I may generate the following organoleptic nitriles:
dodecanenitrile*
tetradecanenitrile*
5-methyl-7-(1-methylethyl)-bicyclo[2.2.2]oct-5-ene-2-carbonitrile*
1,2,3,4-tetrahydro-4,4-dimethyl-1-naphthalenecarbonitrile*
2,4-dimethyl-cyclohex-3-ene-1-nitrile
2,2,3-trimethyl-3-cyclopentene-1-acetonitrile
4-(1,1-dimethylethyl)-benzeneacetonitrile
3,12-tridecadienenitrile
3-methyl-octanenitrile
3-methyl-dodecanenitrile
2-methyl-decanenitrile
decanenitrile
octanenitrile
4,6,6-trimethyl-heptanenitrile
isotetradecanenitrile
3,3-dimethyl-bicyclo[2.2.1]heptane-2-carbonitrile
octahydro-4,7-methano-indene-5-carbonitrile
3,7,7-trimethyl-4-propylidenenitrile-bicyclo[4.1.0] heptane
3-hexyloxy-propionitrile
3-(3,7-dimethyl-oct-2,6-dienyloxy)-propionitrile*
5-cyano-pentan-2-one*
3-methyl-5-cyano-pentan-2-one
4-cyano-2,2-dimethyl-butanal
2-(2-cyanoethyl)-cyclohexanone
3,7-dimethyl-octa-2,6-diennitrile*
3-methyl-5-phenyl-pent-2-enenitrile
3,7-dimethyl-oct-6-enenitrile*
1,2,3,4,5,6,7,8-octahydro-8,8-dimethyl-2-napthalenecarbonitrile
3-methyl-2(3)-nonenenitrile
2-propyl-2-hexenenitrile
4-methyl-2-propyl-hex-2-enenitrile
tridec-2-enenitrile
3,7-dimethyl-nona-2,6-diennitrile*
2,3-dimethyl-2-nonenenitrile
2-undecenenitrile*
cyclohexylidene-phenyl-acetonitrile*
whereby * indicates the preferred nitriles.

Compounds of formula I may generate the following preferred organoleptic oximes:
1,5-dimethyl-bicyclo[3.2.1]octan-8-one oxime
2,4,4,7-tetramethylnona-6,8-dien-3-one oxime
5-methyl-heptan-3-one oxime
1-bicyclo[2.2.1]hept-5-en-2-yl-ethanone oxime

It is a matter of course, that it is not possible to give a complete list of the organoleptic and/or antimicrobial aryl and heteroaryl ketones, ketones, aldehydes, alcohols, phenols, amines, nitriles, thiols, oximes and lactones which are generated as a result of the desired cleavage of the ketones of the formula I. The skilled person is, however, quite aware of those substances which provide the desired organoleptic, e.g. fragrance and odour masking and/or antimicrobial effects.

The compounds of the formula I may preferably be used as sustained release odorants but also as sustained agents to mask or attenuate undesirable odours or to provide additional odours initially present in consumer products, i.e. laundry detergents, fabric softeners, fabric softener sheets, cleaning compositions, swimming pool additives, toiletries and cosmetic products such as shampoo, conditioners and sunscreens. Further applications are sustained antimicrobial agents in the same products.

The amount required to produce the desired, overall effect varies depending upon the particular compounds of formula I chosen, the product in which it will be used, and the particular effect desired.

For example, depending upon the selection and concentration of the compound chosen, when a compound of the formula I is added either singly or as a mixture, e.g. to a laundry product composition at levels ranging from about 0.001 to about 10 % by weight, an odorant, i.e. an odoriferous aryl ketone, ketone, aldehyde, alcohol, phenol, amine, lactone, thiol, oxime, nitrile or two or three of these odoriferous substances in an organoleptically effective amount is/are released when the product is used. These newly formed odorant(s) serve to enhance the odour of the fragrance. Depending on the compound of formula I an antimicrobial agent can be released.

As is evident from the above compilation of organoleptic substances, a broad range of known odorants can be generated from precursors of the present invention. While manufacturing compositions the precursors of the present invention may be used according to methods known to the perfumer, such as e.g. from W.A. Poucher, Perfumes, Cosmetics, Soaps, 2, 7th Edition, Chapman and Hall, London 1974.

The compounds of formula I can be prepared by using standard methods known to the skilled chemist. A wide variety of methods for their preparation is known.

Convenient methods are outlined in the Examples without limiting the invention thereto.

### Example 1

### p-Methoxyphenacyl acetate

To a solution of 63 g potassium acetate in 750 ml of methanol, 92.9 g of 4-methoxyphenacyl bromide was dropped in. The resulting solution was refluxed for 4 hours, then cooled and poured into water. The aqueous layer was extracted with ether, then the combined organic phases were washed with water, dried and evaporated to dryness. The resulting solid was recrystallized from methanol to yield 54.5 g crystals.
- NMR (CDCl₃) δ: 8.05-7.70 (m, 2H), 7.10-6.70 (m, 2H), 5.30 (s, H), 3.90 (s, 3H), 2.20 (s, 3H)ppm.

### Example 2

### p-Tolyloxy-acetic acid 2-oxo-2-phenyl-ethyl ester

According to the procedure of Example 1, p-tolyloxy-acetic acid 2-oxo-2-phenyl-ethyl ester was prepared from (4-methylphenoxy) acetic acid and 2-bromoacetophenone.

### Example 3

### 2-Amino-3-(3,4-dihydroxy-phenyl)-propionic acid 2-oxo-2-phenyl-ethyl ester

According to the procedure of Example 1, 2-amino-3-(3,4-dihydroxy-phenyl)-propionic acid 2-oxo-2-phenyl-ethyl ester was prepared from 3-(3,4-dihydroxy phenyl)alanine and 2-bromoacetophenone.

### Example 4

### Acetic acid 1-methyl-2-oxo-2-phenyl-ethyl ester

According to the procedure of Example 1, acetic acid 1-methyl-2-oxo-2-phenyl-ethyl ester was prepared from acetic acid and 2-bromo-1-phenyl-1-propan-1-one.

### Example 5

### Naphthalen-2-yloxy)-acetic acid 2-(4-methoxy-phenyl)-2-oxo-ethyl ester

According to the procedure of Example 1, (naphthalen-2-yloxy)-acetic acid 2-(4-methoxy-phenyl)-2-oxo-ethyl ester was prepared from (2-naphthoxy)acetic acid and 4-methoxy-phenacyl bromide.

### Example 6

### Cyclopropane carboxylic acid 2-naphthalen-2-yl-2-oxo-ethyl ester

According to the procedure of Example 1, cyclopropane carboxylic acid 2-naphthalen-2-yl-2-oxo-ethyl ester was prepared from cyclopropane carboxylic acid and 2-bromo-1-naphthalen-2-yl-ethanone.

### Example 7

### Acetic acid 2-(3-ethyl-5,5,8,8-tetramethyl-5,6,7,8-tetrahydronaphthalen-2-yl)-2-oxo-ethyl ester

According to the procedure of Example 1, acetic acid 2-(3-ethyl-5,5,8,8-tetramethyl-5,6,7,8-tetrahydro-naphthalen-2-yl)-2-oxo-ethyl ester was prepared from acetic acid and 2-bromo-1-(3-ethyl-5,5,8,8-tetramethyl-5,6,7,8-tetrahydronaphthalen-2-yl)-ethanone.

### Example 8

### 3-(3-Isopropylphenyl)butanol

To a refluxing solution of 132.8 g aluminum isopropoxide in 800 ml of isopropanol, a solution of 300 g 3-(3-isopropylphenyl)butanal in 200 ml of isopropanol was dropped in. The resulting solution was refluxed for 1.5 hours, then cooled down and diluted with ether. Then the organic phase was washed with brine, dried and evaporated to dryness. The resulting oil was distilled to yield 223.5 g of a colourless oil.
- NMR (CDCl₃) δ: 7.30-6.96 (m, 4H), 3.69-3.43 (t, 2H), 2.99-2.73 (m,2H), 1.92-1.77 (m, 2H), 1.60-1.44 (br s, OH), 1.39-1.19 (m, 9H)ppm.

### Example 9

### Octyloxy-acetonitrile

To a suspension of 15.60 g sodium hydride (60%) in 200 ml of 1,2-dimethoxyethane, 50.79 g 1-octanol was dropped in and then the mixture was stirred for 45 min. at room temperature. Then 46.76 g bromoacetonitrile was dropped in, the reaction mixture was refluxed for 2 hours, quenched with water, filtrated and diluted with brine. After extraction with ether, the organic layer was washed with brine and water, dried, filtered and evaporated to dryness. The resulting dark oil was purified by distillation to yield a yellow oil.
- NMR (CDCl₃) δ: 4.24 (s, 2H), 3.63-3.52 (t, 2H), 1.75-1.50(m, 2H), 1.49-1.15 (m, 10H), 0.96-0.80 (t, 3H)ppm.

### Example 10

### Undec-10-enyloxy-acetonitrile

According to the procedure of Example 9, undec-10-enyloxy-acetonitrile was prepared from undec-10-en-1-ol, bromoacetonitrile and sodium hydride.

### Example 11

### [3-(3-Isopropyl-phenyl)-butoxy]-acetonitrile

According to the procedure of Example 9, [3-(3-isopropylphenyl)-butoxy]-acetonitrile was prepared from 3-(3-isopropylphenyl)butanol, bromoacetonitrile and sodium hydride.

### Example 12

### [1-Methyl-3-(2,6,6-trimethyl-cyclohex-1-enyl)-allyloxy]-acetonitrile

According to the procedure of Example 9, [1-methyl-3-(2,6,6-trimethyl-cyclohex-1-enyl)-allyloxy]-acetonitrile was prepared from 4-(2,6,6-trimethyl-cyclohex-1-enyl)but-3-en-2-ol (Marvell et al., J. Org. Chem., 37 (1972) 19, 2992-97), bromoacetonitrile and sodium hydride.

### Example 13

### (2-Pentyl-3-phenyl-allyloxy)-acetonitrile

According to the procedure of Example 9, (2-pentyl-3-phenyl-allyloxy)-acetonitrile was prepared from amyl cinnamic alcohol, bromoacetonitrile and sodium hydride.

### Example 14

### 2-(3,7-Dimethyl-oct-6-enyloxy)-1-naphthalen-2-yl-ethanone

To a suspension of 2.24 g magnesium in ether, a solution of 19.09 g 2-bromonaphthalene in 80 ml of ether was dropped in and then the mixture was heated to reflux. After refluxing for one hour, the mixture was cooled down to 0-5°C and a solution of 15.00 g (3,7-dimethyl-oct-6-enyloxy)-acetonitrile (Kulka, K. et al., Perfumer & Flavorist Vol. 3 (1978), p.39) in 20 ml of ether was dropped in at this temperature. After 3 hours stirring at room temperature, the mixture was quenched with water and aqueous sulfuric acid. After extracting with ether, the combined organic phases were washed with saturated sodium bicarbonate and water, dried, filtered and evaporated to dryness. The resulting dark oil was purified by chromatography to yield 15.22 g of a yellow oil.
- NMR (CDCl₃) δ: 8.49 (s, 1H), 8.05-7.79 (m, 4H), 7.67-7.42 (m, 2H), 5.15-5.01 (t, 1H), 4.83 (s, 2H), 3.73-3.55 (t, 2H), 2.11-1.83, (m, 2H), 1.82-1.03 (m, 11H), 0.96-0.81 (d, 3H)ppm.

### Example 15

### 2-Ethoxy-1-phenyl-ethanone

According to the procedure of Example 12, 2-ethoxy-1-phenylethanone was prepared from bromobenzene and ethoxyacetonitrile.

### Example 16

### 1-Naphthalen-2-yl-2-octyloxy-ethanone

According to the procedure of Example 12, 1-naphthalen-2-yl2-octyloxy-ethanone was prepared from 2-bromonaphthalene and octyloxy-acetonitrile.

### Example 17

### 2-(3,7-Dimethyl-oct-6-enyloxy)-1-(4-methoxy-phenyl)-ethanone

According to the procedure of Example 12, 2-(3,7-Dimethyl-oct-6-enyloxy)-1-(4-methoxy-phenyl)-ethanone was prepared from 4-bromoanisole and (3,7-dimethyl-oct-6-enyloxy)-acetonitrile (Kulka, K. et al., Perfumer & Flavorist Vol. 3 (1978), p.39).

### Example 18

### 2-(3,7-Dimethyl-oct-6-enyloxy)-1-(4-hydroxy-phenyl)-ethanone

According to the procedure of Example 12, 2-(3,7-Dimethyl-oct-6-enyloxy)-1-(4-hydroxy-phenyl)-ethanone was prepared from (4-bromo-phenoxy)-trimethyl-silane (J. Org. Chem., 48 (1983), 1543) and (3,7-dimethyl-oct-6-enyloxy)-acetonitrile (Kulka, K. et-al., Perfumer & Flavorist Vol. 3 (1978), p.39).

### Example 19

### 1-Naphthalen-2-yl-2-undec-10-enyloxy-ethanone

According to the procedure of Example 12, 1-naphthalen-2-yl-2-undec-10-enyloxy-ethanone was prepared from 2-bromonaphthalene and undec-10-enyloxy-acetonitrile.

### Example 20

### 2-[3-(3-Isopropyl-phenyl)-butoxy]-1-naphthalen-2-yl-ethanone

According to the procedure of Example 12, 2-[3-(3-isopropylphenyl)-butoxy]-1-naphthalen-2-yl-ethanone was prepared from 2-bromonaphthalene and [3-(3-isopropyl-phenyl)-butoxy]acetonitrile.

### Example 21

### 2-[3-(3-Isopropyl-phenyl)-butoxy]-1-(4-methoxy-phenyl)-ethanone

According to the procedure of Example 12, 2-[3-(3-isopropyl-phenyl)-butoxy]-1-(4-methoxy-phenyl)-ethanone was prepared from 4-bromoanisole and [3-(3-isopropyl-phenyl)-butoxy]-acetonitrile.

### Example 22

### 2-(3,7-Dimethyl-oct-6-enyloxy)-1-phenantren-9-yl-ethanone

According to the procedure of Example 12, 2-(3,7-dimethyl-oct-6-enyloxy)-1-phenantren-9-yl-ethanone was prepared from 9-bromo-phenantrene and (3,7-dimethyl-oct-6-enyloxy)-acetonitrile (Kulka, K. et al., Perfumer & Flavorist Vol. 3 (1978), p.39).

### Example 23

### 1-(4-Methoxy-phenyl)-2-[1-methyl-3-(2,6,6-trimethyl-cyclohex-1-enyl)-allyloxy]-ethanone

According to the procedure of Example 12, 1-(4-methoxy-phenyl)-2-[1-methyl-3-(2,6,6-trimethyl-cyclohex-1-enyl)-allyloxy]-ethanone was prepared from 4-bromoanisole and [1-methyl-3-(2,6,6-trimethyl-cyclohex-1-enyl)-allyloxy]-acetonitrile.

### Example 24

### 1-(4-Methoxy-phenyl)-2-(2-pentyl-3-phenyl-allyloxy)-ethanone

According to the procedure of Example 12, 1-(4-methoxy-phenyl)-2-(2-pentyl-3-phenyl-allyloxy)-ethanone was prepared from 4-bromoanisole and (2-pentyl-3-phenyl-allyloxy)-acetonitrile.

### Example 25

### Hydroxy-acetic acid 2-oxo-2-phenyl-ethyl ester

A mixture of 7.61 g glycolic acid, 19.90 g phenacylbromide and 13.9 ml triethylamine in 300 ml of ethyl acetate was stirred for 20 hours at room temperature. Then the mixture was filtered and the filtrate was washed with 2N HCl, saturated sodium bicarbonate and brine, dried, filtered and evaporated to dryness. The resulting white solid was purified by recrystallisation to yield 11.85 g of colourless crystals.
- NMR (CDCl₃) δ: 7.97-7.86 (m, 2H), 7.71-7.44 (m, 3H), 5.49 (s, 2H), 4.45-4.36 (d, 2H), 2.59-2.47 (t, 1H)ppm.

### Example 26

### Carbonic acid 4-allyl-2-methoxy-phenyl ester 2-(4-methoxy-phenyl)-2-oxo-ethyl ester

To a solution of 17.62 g 2-hydroxy-4'-methoxyacetophenone (Kovach et al., J. Amer. Chem. Soc., Vol. 115, No. 23, (1993) p. 10476) and 12.96 g pyridine in 240 ml of dichloromethane, a solution of 26.32 g eugenol-chloroformate in 60 ml of dichloromethane was dropped in at 5-10°C and then the mixture was stirred for 6 hours at room temperature. Then the reaction mixture was acidified with 2N HCl and extracted with ether. The combined organic phases were washed with 2N HCl, saturated sodium bicarbonate and brine, dried, filtered and evaporated to dryness. The resulting solid was purified by recrystallisation to yield 29.79 g of yellow crystals.
- NMR (CDCl₃) δ: 7.98-7.86 (m, 2H), 7.20-6.72 (m, 5H), 6.09-5.83 (m, 1H), 5.41 (s, 2H), 5.18-5.02 (m, 2H), 3.89 (s, 3H), 3.87 (s,3H), 3.43-3.32 (d, 2H)ppm.

### Example 27

### Carbonic acid 3-methyl-5-phenyl-pentyl ester 2-naphthalen-2-yl-2-oxo-ethyl ester

According to the procedure of Example 22, carbonic acid 3-methyl-5-phenyl-pentyl ester 2-naphthalen-2-yl-2-oxo-ethyl ester was prepared from 2-hydroxy-1-naphthalen-2-yl-ethanone (Langenbeck et al., Chem. Ber., 69 (1936) 514-516), 3-methyl-5-phenyl-pentanol-chloroformate and pyridine.

### Example 28

### 4-Hydroxy-undecanoic acid sodium salt

To a solution of 43.6 g sodium hydroxide in 150 ml of methanol heated to reflux, 200 g gamma-undecalactone were dropped in. After stirring 2 hours at reflux, the mixture was cooled to room temperature and evaporated to dryness. The resulting crystals were washed with hexane to yield 240 g of colourless crystals.
- NMR (CDCl₃) δ: 5.1-4.8 (br s, OH), 3.63-3.42 (m, 1H), 2.39-2.20 (t,2H), 1.89-1.52(m, 2H), 1.51-1.15 (m, 12H), 1.00-0.81, (t, 3H) ppm.

### Example 29

### 4-Hydroxy-undecanoic acid-2-naphthalen-2-yl-2-oxo-ethyl ester

A suspension of 20.00 g 2-bromo-2'-acetonaphtone, 18.01 g 4-hydroxy-undecanoic acid sodium salt and 0.5 g tetrabutylammonium bromide in 180 ml of dimethylformamide was stirred for 6 hours at 50°C. Then the reaction mixture was diluted with ether and washed with water, 2N HCl, saturated sodium bicarbonate and brine, dried, filtered and evaporated to dryness. The resulting solid was purified by recrystallisation to yield 7.92 g of colourless crystals.
- NMR (CDCl₃) δ: 8.41 (s, 1H), 8.03-7.78 (m, 4H), 7.70-7.48 (m, 2H), 5.55-5.42 (2s, 2H), 3.80-3.61 (m, 1H), 2.27-2.60 (t, 2H), 2.33-2.06 (br s, OH), 2.05-1.11 (m, 14H), 1.02-0.79 (t, 3H)ppm.

### Example 30

### 4-Hydroxy-undecanoic acid 2-(4-methoxy-phenyl)-2-oxo-ethyl ester

According to the procedure of Example 24, 4-hydroxy-undecanoic acid 2-(4-methoxy-phenyl)-2-oxo-ethyl ester was prepared from 2-bromo-4'-methoxyacetophenone, 4-hydroxy-undecanoic acid sodium salt and tetrabutylammonium bromide.

### Example 31

### 4-Hydroxy-undecanoic acid 1-methyl-2-oxo-2-phenyl-ethyl ester

According to the procedure of Example 24, 4-hydroxy-undecanoic acid 1-methyl-2-oxo-2-phenyl-ethyl ester was prepared from 2-bromopropiophenone, 4-hydroxy-undecanoic acid sodium salt and tetrabutylammonium bromide.

### Example 32

### 4-(3-Methyl-5-phenyl-pentyloxycarbonyloxy)-undecanoic acid 2-naphthalen-2-yl-2-oxo-ethyl ester

To a solution of 4.00 g 4-hydroxy-undecanoic acid-2-naphthalen-2-yl-2-oxo-ethyl ester and 1.71 g pyridine in 20 ml of THF, a solution of 2.91 g 3-methyl-5-phenyl-pentanol-chloroformate in 10 ml of THF was dropped in at 0°C and then the mixture was stirred for 3 hours at room temperature. Then the reaction mixture was quenched with water and extracted with ether. The organic phase was washed with 2N HCl, saturated sodium bicarbonate and water, dried, filtered and evaporated to dryness. The resulting yellow oil was purified by chromatography to yield 5.46 g of a colorless oil.
- NMR (CDCl₃) δ: 8.42 (s, 1H), 8.03-7.80 (m, 4H), 7.70-7.45 (m, 2H), 7.35-7.03(m, 5H), 5.49 (s, 2H), 4.89-4.71
(m, 1H), 4.30-4.07 (m, 2H), 2.80-2.45(m, 4H), 2.21-1.12 (m, 19H), 1.10-0.78 (m,6H)ppm.

### Example 33

### 4-Hex-3-enyloxycarbonyloxy-undecanoic acid 2-(4-methoxy-phenyl)-2-oxo-ethyl ester

According to the procedure of Example 26, 4-hex-3-enyloxycarbonyloxy-undecanoic acid 2-(4-methoxy-phenyl)-2-oxoethyl ester was prepared from 4-hydroxy-undecanoic acid 2-(4-methoxy-phenyl)-2-oxo-ethyl ester, cis-hex-3-enol-chloroformate and pyridine.

### Example 34

### 4-(3-Methyl-5-phenyl-pentyloxycarbonyloxy)-undecanoic acid 1-methyl-2-oxo-2-phenyl-ethyl ester

According to the procedure of Example 26, 4-(3-methyl-5-phenyl-pentyloxycarbonyloxy)-undecanoic acid 1-methyl-2-oxo-2-phenyl-ethyl ester was prepared from 4-hydroxy-undecanoic acid 1-methyl-2-oxo-2-phenyl-ethyl ester, 3-methyl-5-phenyl-pentanol-chloroformate and pyridine.

### Example 35

### 4-[2-Ethyl-4-(2,2,3-trimethyl-cyclopent-3-enyl)-but-2-enyloxycarbonyloxy]-undecanoic acid 1-methyl-2-oxo-2-phenyl-ethyl ester

According to the procedure of Example 26, 4-[2-ethyl-4-(2,2,3-trimethyl-cyclopent-3-enyl)-but-2-enyloxycarbonyloxy]-undecanoic acid 1-methyl-2-oxo-2-phenyl-ethyl ester was prepared from 4-hydroxy-undecanoic acid 1-methyl-2-oxo-2-phenyl-ethyl ester, 2-ethyl-4-(2,2,3-trimethyl-cyclopentyl-3-en-1-yl)-but-2-en-1-ol chloroformate and pyridine.

### Example 36

### 4-[1,2-Dimethyl-4-(2,2,3-trimethyl-cyclopent-3-enyl)-but-3-enyloxycarbonyloxy]-undecanoic acid 1-methyl-2-oxo-2-phenyl-ethyl ester

According to the procedure of Example 26, 4-[1,2-dimethyl-4-(2,2,3-trimethyl-cyclopent-3-enyl)-but-3-enyloxycarbonyloxy]-undecanoic acid 1-methyl-2-oxo-2-phenyl-ethyl ester was prepared from 4-hydroxy-undecanoic acid 1-methyl-2-oxo-2-phenyl-ethyl ester, 3-methyl-5-(2,2,3-trimethyl-3-cyclopenten-1-yl)-4-penten-2-ol chloroformate and pyridine.

### Example 37

Test cloth was washed with detergent to which one or more of the precursors of Examples 1-7, 12-22, 24-26 had been added. The cloth was then line dried. The cloth dried in sunlight had a distinct fragrance note, as determined by a trained panel. In contrast, the cloth dried without light was olfactively neutral.

### Example 38

Test cloth was washed with a detergent and then a fabric softener, containing one or more of the precursors of Examples 1-7, 12-22, 24-26 was added to the rinse cycle. The cloth was then line dried. The cloth dried in sunlight had a distinct fragrance note, as dertermined by a trained panel. In contrast, the cloth dried without sunlight was olfactively neutral.

### Example 39

### Preparation of an O/W sunscreen lotion UV-B and UV-A:

Sunscreen lotion containing 0.5 % of one or more compounds of Examples 1-7, 12-22, 24-26.

| Recipe: % | **compound** |
|---|---|
| **Part A:** | |
| 2 % | Octyl methoxycinnamate |
| 3 % | 4-4-Butyl-4'methoxydibenzoyl methane |
| 12 % | Coco-caprylate/caprate |
| 4 % | Isostearyl neopentanoate |
| 0.25 % | Diethyleneglycol monostearate |
| 1 % | Cetylalcohol |
| 0.25 % | Methyl-propylparabene |
| 0.1 % | EDTA-sodium salt |
| 1 % | Diethanolamine cetylphosphate |

| **Part B:** | |
|---|---|
| 20 % | Acrylate C10-C30 Alkylacrylate |
| 50.1 % | water deion. |
| 5 % | 1,2-Propanediol |
| 0.8 % | Potassium hydroxide. |
| Part A was heated in a reactor to 85°C. | |
| Part B was slowly added within 10 min., followed by addition of KOH and 0.5 % of one or more of the compounds in Examples 1-7, 12-22, 24-26. The emulsion was then cooled and degassed. | |

### Example 40

a) Fabric softener of the ester quat type (4 x concentrate):

| INGREDIENTS CHEMICAL NAME | % |
|---|---|
| PHASE A | |
| DEIONISED WATER | to 100.0 |
| | |
| MgCl₂ (saturated sol.)Magnesium chloride | 1.0 |
| | |

| PHASE B | |
|---|---|
| REWOQUAT WE 18Di-(tallow carboxyethyl)hydroxy ethyl methylammonium methosulfate | 15.0 |
| | |
| GENAPOL O 100Ethoxylated fatty alcohol C16-C18 10EO | 2.0 |
| | |
| ANTIFOAM DB 31 | 0.5 |
| | |

| PHASE C | |
|---|---|
| ISOPROPYL ALCOHOL | 3.0 |
| | |
| PRESERVATIVE | Qs |
| | |
| PERFUME | Qs |

PROCESS:
While stirring and heating to 65° C, mix part A, then part B preheated to 65° C. After cooling to room temperature, add part C. The pH value of the finished product is 2.60. Recommended level of perfume is 1.0 %. Delayed release fragrances of Examples 1-7, 12-22, 24-26 may be any part of this 1.0 %.
b)Fabric softener of the ester quat type (1 x concentrate):

| INGREDIENTS CHEMICAL NAME | % |
|---|---|
| PHASE A | |
| DEIONISED WATER | to 100.0 |
| | |

| PHASE B | |
|---|---|
| REWOQUAT WE 18Di-(tallow carboxyethyl)hydroxy ethyl methylammonium methosulfate | 6.0 |
| | |
| DOBANOL 25-9Ethoxylated fatty alcohol C12-C15 9EO | 0.50 |
| | |
| ANTIFOAM DB 31 | 0.10 |
| | |

| PHASE C | |
|---|---|
| MYACIDE BT 302-bromo-2-nitropropane 1,3 diol | 0.03 |
| | |
| PROXEL GXLBenzisothiazolinone sodium salt | 0.02 |
| | |
| PERFUME | Qs |

PROCESS:
While stirring and heating to 65° C, mix part A, then part B preheated to 65° C. After cooling to room temperature, add part C. The pH value of the finished product is 3.50.
Recommended level of perfume: 0.3 %. Delayed release fragrances of Examples 1-7, 12-22, 24-26 may be any part of this 0.3 %.

## Claims

1. Ketones of the formula I 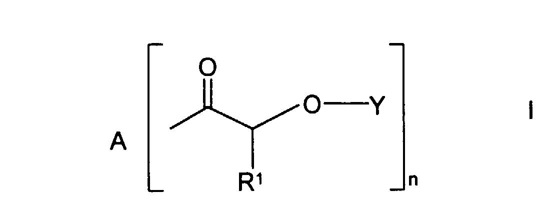 wherein
Y is an optionally substituted alkyl, cycloalkyl, cycloalkylalkyl, alkylcarbonyl, cycloalkylcarbonyl, arylcarbonyl, alkyloxycarbonyl, cycloalkyloxycarbonyl, aryloxycarbonyl, amide, oximinocarbonyl, alkylthiocarbonyl, cycloalkylthiocarbonyl, whereby all alkyl groups may be straight or branched and all alkyl and cycloalkyl groups may be saturated or unsaturated,
R¹ is hydrogen or a C₁₋₆ alkyl group which may be substituted, saturated or unsaturated, straight or branched,
A is a chromophoric aromatic or heteroaromatic ring or ring system,
A may carry 1 to 8 substituents selected from hydrogen, hydroxy, mercapto, amino, acyl, ester, esterified hydroxy group, cyano and nitro; alkyl, alkoxy, alkylthio and arylalkoxy which may be substituted, saturated or unsaturated, straight or branched; aryl and heteroaryl which may be substituted; one of the substituents may be a polymeric group, and
n is an integer
with the exception of
2-ethoxy-1-phenyl-ethanone,
hydroxy-acetic acid 2-oxo-2-phenyl-ethyl ester,
acetic acid 1-methyl-2-oxo-2-phenyl-ethyl ester, and p-methoxyphenacyl acetate.

2. Ketones according to claim 1, which yield after cleavage by light one or more organoleptic compounds.

3. Ketones according to claim 1 or 2, which yield after cleavage by heat one or more organoleptic compounds.

4. Ketones according to any of claims 1 to 3, which yield after cleavage by hydrolysis one or more organoleptic compounds.

5. Ketones according to any of claims 1 to 4, which yield after cleavage by enzymes one or more organoleptic compounds.

6. Ketones according to any of claims 1 to 5, which yield after cleavage two or more organoleptic compounds.

7. Ketones according to any of claims 1 to 6, which yield after cleavage an organoleptic aryl ketone of the formula II

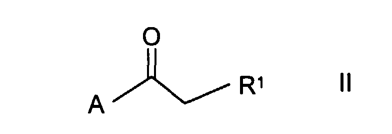

8. Ketones according to any of claims 1 to 7, which yield after cleavage one or more organoleptic aldehydes or ketones derived from O-Y.

9. Ketones according to any of claims 1 to 7, which yield an organoleptic alcohol, phenol, aldehyde or ketone derived from Y.

10. Ketones according to any of claims 1 to 7, which yield after cleavage an organoleptic lactone derived from Y.

11. Ketones according to any of claims 1 to 7, which yield after cleavage an amine used in perfumery and derived from Y.

12. Ketones according to any of claims 1 to 11, wherein R¹ is hydrogen or methyl.

13. Ketones according to any of claims 1 to 12, wherein R¹ is hydrogen or methyl and A carries no more than three substituents, preferably methyl groups.

14. Ketones according to any of claims 1 to 12, wherein R¹ is hydrogen and A is one aromatic ring which is substituted by one methoxy group, preferably in the para-position.

15. Ketones according to any of claims 1 to 13, wherein A is a ring system of the formula 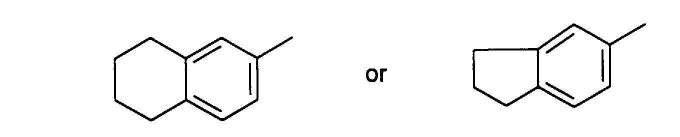 and both rings may be substituted preferably with one or more methyl groups.

16. Ketones according to any of claims 1 to 15, wherein n is 1 or 2.

17. Ketones according to any of claims 1 to 16, wherein one of the substituents of A is a polymeric group.

18. Ketones to any of the claims 1 to 17, wherein the groups for which Y stands are substituted with one or more substituents selected from aryl; cycloalkyl;
cycloalkenyl; oxo; oxy; cyano; amino; hydroxy; hydroxy being esterified with a carboxylic acid, a carbonic acid ester (carbonate), a N,N-substituted carbamic acid (carbamate) or an oximino carbonic acid; amino being substituted with oximinocarbonyl; an esterified carboxylic group; and a carboxylic group esterified with an oxime.

19. Use of ketones of formula I 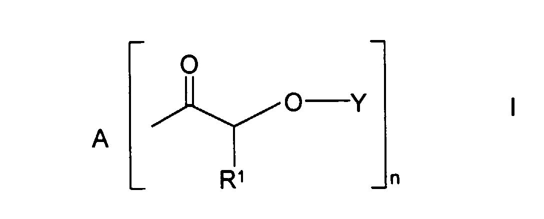 wherein
Y is an optionally substituted alkyl, cycloalkyl, cycloalkylalkyl, alkylcarbonyl, cycloalkylcarbonyl, arylcarbonyl, alkyloxycarbonyl, cycloalkyloxycarbonyl, aryloxycarbonyl, amide, oximinocarbonyl, alkylthiocarbonyl, cycloalkylthiocarbonyl, whereby all alkyl groups may be straight or branched and all alkyl and cycloalkyl groups may be saturated or unsaturated,
R¹ is hydrogen or a C₁₋₆ alkyl group which may be substituted, saturated or unsaturated, straight or branched,
A is a chromophoric aromatic or heteroaromatic ring or ring system,
A may carry 1 to 8 substituents selected from hydrogen, hydroxy, mercapto, amino, acyl, ester, esterified hydroxy group, cyano and nitro; alkyl, alkoxy, alkylthio and arylalkoxy which may be substituted, saturated or unsaturated, straight or branched; aryl and heteroaryl which may be substituted; one of the substituents may be a polymeric group, and
n is an integer,
as precursors.

20. Use according to claim 19 as precursors for fragrances and/or antimicrobial agents.

21. Use according to claim 20 in detergents, fabric softeners, personal care products, cleaning compositions, cosmetics and sun protection products.
